# EUROPEAN PATENT SPECIFICATION

(11) **EP 3 716 897 B1**
(45) Date of publication and mention of the grant of the patent: **11.10.2023**
(21) Application number: 17933691.2
(22) Date of filing: 30.11.2017
(51) Int. Cl.: A61F 2/00, A61F 2/01, A61F 2/04, A61F 2/06

(54) **EMBOLIC PROTECTION DEVICE AND SYSTEM**
EMBOLIESCHUTZVORRICHTUNG UND SYSTEM
DISPOSITIF ET SYSTÈME DE PROTECTION EMBOLIQUE

(43) Date of publication of application: 07.10.2020
(73) Proprietor: C. R. Bard, Inc., Franklin Lakes, NJ 07417 (US)
(72) Inventor: RANDALL, Scott L., Tempe, AZ 85280 (US)
(74) Representative: Hoffmann Eitle
(86) International application number: PCT/US2017/064046
(87) International publication number: WO 2019/108212

(56) References cited:
- US-A1- 2003 065 356
- US-A1- 2006 074 474
- US-A1- 2008 065 008
- US-A1- 2010 185 179
- US-A1- 2010 318 097
- US-A1- 2014 207 178
- US-A1- 2015 223 829
- US-A1- 2017 112 647

## Description

### BACKGROUND

Atherosclerosis is characterized by one or more intravascular lesions formed, in part, of plaque including blood-borne substances such as fat, cholesterol, or calcium. An intravascular lesion such as an arterial lesion can form on a wall of an arterial lumen and build out across the lumen to an opposite arterial wall. A last point of patency often occurs between the arterial lesion and the opposite arterial wall.

Peripheral artery disease is characterized by one or more peripheral arteries such as one or more arteries of the arms or the legs being narrowed or blocked by arterial lesions. The narrowed or blocked arteries can result in ischemia or a loss of blood flow to such peripheral arteries, complications from which can include bacterial infection, infarction or tissue necrosis, or both. If not timely addressed, such complications can require limb amputation.

Accepted surgical intervention for peripheral artery disease includes balloon angioplasty and stenting with balloon-expandable stents, self-expanding stents, or stent grafts to restore patency and blood flow impeded by arterial lesions. More recently, atherectomy has been shown to be an effective alternative surgical intervention to restore patency and blood flow with less vessel trauma. However, embolization of emboli such as calcifications, tissue, or other debris resulting from crossing or ablating the arterial lesions remains a concern in each of the foregoing surgical interventions. Provided herein, in some embodiments, are embolic protection devices, as well as systems and methods thereof that address at least the foregoing. US-A-2006/0047301 discloses a catheter-based emboli-removal system. US 2003/0065356 discloses a catheter-based emboli-filtering apparatus. US 2017/112647 discloses a percutaneous angioplasty device including a filter coupled to a catheter.

### SUMMARY

According to the invention there is provided a catheter assembly according to claim 1. The catheter body is sized for endoluminal advancement of a distal end portion of the catheter assembly from a puncture site at a human artery selected from a dorsalis pedis artery, a fibular artery, an anterior tibial artery, and a posterior tibial artery toward one or more larger arteries. The catheter assembly has an insertion profile when the embolic protection device is in a collapsed state, and a deployment profile when the embolic protection device is in a deployed state. The embolic protection device assumes the collapsed state while stowed in the catheter-body lumen at the distal end portion of the catheter assembly. The embolic protection device assumes the deployed state while outside the catheter-body lumen. The deployed state of the embolic protection device is configured with a retrograde opening away from the catheter body and toward a distal end or distal cap of an arterial lesion for capturing emboli dislodged from an arterial lesion while at a distal end of the arterial lesion. The dependent claims are directed to optional features and preferred embodiments.

In some embodiments, the catheter body is sized at about 3 Fr (1 mm) or less.

In some embodiments, the embolic protection device includes a mesh or a porous membrane.

In some embodiments, the embolic protection device includes non-porous membrane.

In some embodiments, the catheter assembly further includes a control mechanism configured to deploy the embolic protection device. The control mechanism includes a control selected from a slide button and a scroll wheel at a proximal end portion of the catheter assembly. The control mechanism connects to the embolic protection device by one or more wires configured to deploy the embolic protection device upon advancing the control to a deployment position, thereby putting the catheter assembly in the deployment profile with the embolic protection device thereof in the deployed state.

In some embodiments, the catheter assembly is configured for endoluminal advancement of the distal end portion of the catheter assembly over a guidewire.

In some embodiments, the catheter body includes a diameter commensurate with an average diameter of a human dorsalis pedis artery, the diameter obviating endoluminal advancement of the distal end portion of the catheter assembly over a guidewire.

In some embodiments, the distal end portion of the catheter assembly includes one or more radiopaque markings for endoluminal advancement of the catheter assembly using fluoroscopic imaging.

In some embodiments, at least the distal end portion of the catheter assembly is echogenic for endoluminal advancement of the catheter assembly using ultrasound.

The catheter body further includes one or more additional lumens, each lumen of the one or more additional lumens independently configured for flushing an arterial lumen around a distal end of the arterial lesion, aspirating emboli dislodged from the arterial lesion, or both flushing and aspirating in accordance with the foregoing.

In some embodiments, the catheter assembly is coupled to a console configured to control one or more functions of the catheter assembly.

In some embodiments, the console includes a control mechanism including a control selected from a slide button, a scroll wheel, a push button, and a switch on the console configured to deploy the embolic protection device. The control mechanism connects to the embolic protection device by one or more wires configured to deploy the embolic protection device upon advancing the control to a deployment position, thereby putting the catheter assembly in the deployment profile with the embolic protection device thereof in the deployed state.

In some embodiments, the console further includes one or more pumps and a reservoir. The one or more pumps are fluidly connected to at least one of the one or more additional lumens for flushing the arterial lumen around a distal end of the arterial lesion, aspirating emboli dislodged from the arterial lesion, or both flushing and aspirating in accordance with the foregoing. The reservoir is configured for collecting emboli dislodged from the arterial lesion.

It will be appreciated that the invention makes available a catheter system including, a catheter assembly and a console configured to control one or more functions of the catheter assembly. The catheter assembly includes a catheter body with a catheter-body lumen and an embolic protection device. The catheter body is sized for endoluminal advancement of a distal end portion of the catheter assembly from a puncture site at a human artery selected from a dorsalis pedis artery, a fibular artery, an anterior tibial artery, and a posterior tibial artery toward one or more larger arteries. The catheter assembly has an insertion profile when the embolic protection device is in a collapsed state, and a deployment profile when the embolic protection device is in a deployed state. The embolic protection device assumes the collapsed state while stowed in the catheter-body lumen at the distal end portion of the catheter assembly. The embolic protection device assumes the deployed state while outside the catheter-body lumen. The deployed state of the embolic protection device is configured with an opening away from the catheter body for capturing emboli dislodged from an arterial lesion while at a distal end of the arterial lesion. The deployed state of the embolic protection device may take on the shape of a basket, funnel, cone, or the like such that the cross-sectional area of the embolic protection device decreases from the opening to the catheter body following deployment.

In some embodiments, the catheter body is sized at about 3 Fr (1 mm) or less.

In some embodiments, the catheter system further includes a control mechanism including a control selected from a slide button, a scroll wheel, a push button, and a switch on the console configured to deploy the embolic protection device. The control mechanism connects to the embolic protection device by one or more wires configured to deploy the embolic protection device upon advancing the control to a deployment position, thereby putting the catheter assembly in the deployment profile with the embolic protection device thereof in the deployed state.

In some embodiments, the console further includes one or more pumps and a reservoir. The one or more pumps are fluidly connected to at least one of the one or more additional lumens for flushing the arterial lumen around a distal end of the arterial lesion, aspirating emboli dislodged from the arterial lesion, or both flushing and aspirating in accordance with the foregoing. The reservoir is configured for collecting emboli dislodged from the arterial lesion.

### DRAWINGS

FIG. 1 provides a schematic illustrating a catheter assembly including an embolic protection device.
FIG. 2A provides a schematic illustrating a catheter assembly including an embolic protection device in a collapsed state.
FIG. 2B provides a schematic illustrating a catheter assembly including an embolic protection device in a deployed state.
FIG. 3A provides a schematic illustrating a system including a console and a catheter assembly with an embolic protection device in a collapsed state.
FIG. 3B provides a schematic illustrating a system including a console and a catheter assembly with an embolic protection device in a deployed state.
FIG. 3C provides a schematic illustrating an alternative for the catheter assembly of the system of FIGS. 3A and 3B.
FIG. 4A provides a schematic illustrating a system including a console and a control mechanism for an embolic protection device of a catheter assembly.
FIG. 4B provides a schematic illustrating a system including a console and a control mechanism for an embolic protection device of a catheter assembly.
FIG. 4C provides a schematic illustrating a system including a console and a control mechanism for an embolic protection device of a catheter assembly.
FIG. 5 provides a schematic illustrating an arterial lesion impeding blood flow in an artery.
FIG. 6 provides a schematic illustrating arteries in a human leg including the dorsalis pedis artery, the fibular artery, the anterior and posterior tibial arteries, the popliteal artery, and the femoral artery.

### DESCRIPTION

Before some particular embodiments of the invention are provided in greater detail, it should be understood that a particular embodiment provided herein can have features that can be readily separated from the particular embodiment and optionally combined with or substituted for features of any of a number of other embodiments provided herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "front," "back," "top," "bottom," "forward," "reverse," "clockwise," "counter clockwise," "up," "down," or other similar terms such as "upper," "lower," "aft," "fore," "vertical," "horizontal," "proximal," "distal," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter provided herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter provided herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

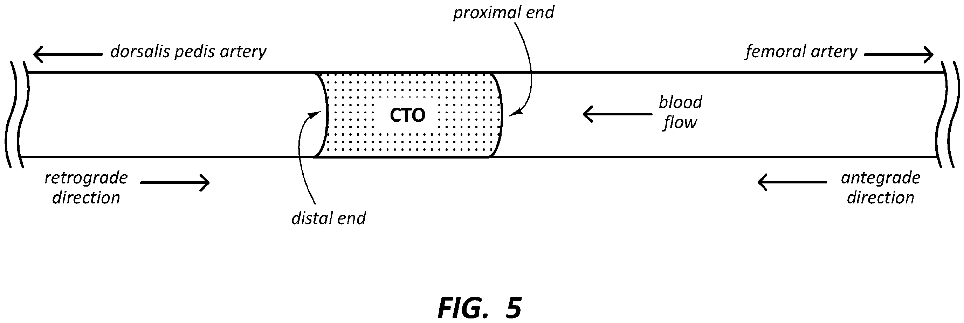

With respect to a distal end or distal end cap of an arterial lesion, FIG. 5 shows the distal end or the distal end cap of the arterial lesion opening or otherwise directed in an antegrade direction with a flow of blood. With respect to a proximal end or proximal end cap of an arterial lesion, FIG. 5 also shows the proximal end or the proximal end cap of the arterial lesion opening or otherwise directed in an retrograde direction against the flow of blood.

Unless defined otherwise, all technical and scientific terms used herein have the same meaning as commonly understood by those of ordinary skill in the art.

Again, accepted surgical intervention for peripheral artery disease includes balloon angioplasty and stenting with balloon-expandable stents, self-expanding stents, or stent grafts to restore patency and blood flow impeded by arterial lesions. More recently, atherectomy has been shown to be an effective alternative surgical intervention to restore patency and blood flow with less vessel trauma. However, embolization of emboli such as calcifications, tissue, or other debris resulting from crossing or ablating the arterial lesions remains a concern in each of the foregoing surgical interventions. Provided herein, in some embodiments, are embolic protection devices, as well as systems and methods thereof that address at least the foregoing.

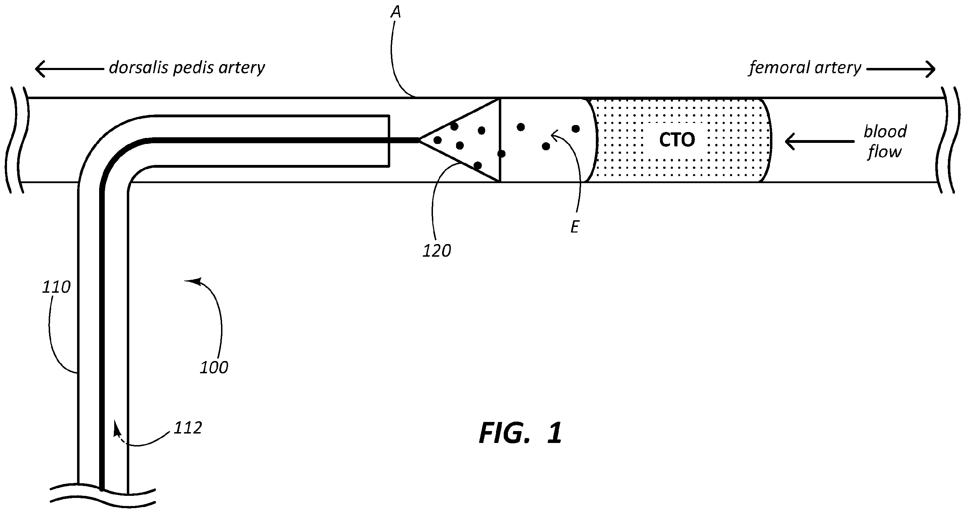

For example, FIG. 1 provides a schematic illustrating a catheter assembly 100 including an embolic protection device 120 in accordance with the invention.

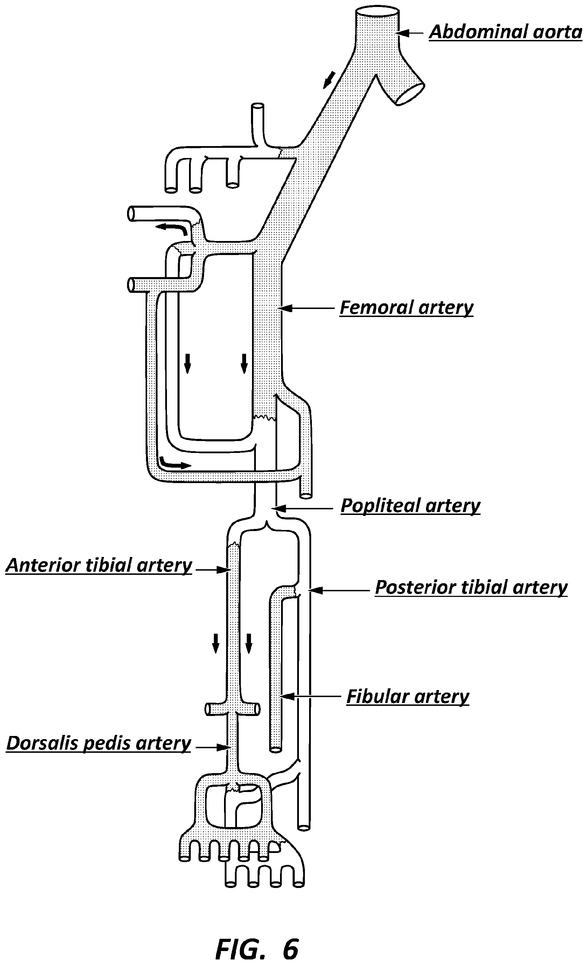

As shown, the catheter assembly 100 includes a catheter body 110 with at least one catheter-body lumen 112 from which catheter-body lumen 112 the embolic protection device 120 is deployed for capturing emboli E. The catheter body 110 is sized for endoluminal advancement of a distal end portion of the catheter assembly 100 from a puncture site at a patient's artery A toward one or more larger arteries. Such endoluminal advancement can be retrograde endoluminal advancement, the puncture site at the patient's artery A being at the dorsalis pedis artery, and the one or more larger arteries being the anterior tibial artery, the popliteal artery, or the femoral artery. FIG. 6 provides a schematic illustrating such arteries in a human leg. The puncture site at the patient' s artery A can alternatively be at the anterior tibial artery, and the one or more larger arteries can be the popliteal artery or the femoral artery. The puncture site at the patient's artery A can alternatively be at the fibular artery, and the one or more larger arteries can be the posterior tibial artery, the popliteal artery, or the femoral artery. The puncture site at the patient's artery A can alternatively be at the posterior tibial artery, and the one or more larger arteries can be the popliteal artery or the femoral artery. Such endoluminal advancement is useful for embolic protection at a distal side of an arterial lesion while treating (e.g., crossing, ablating, etc.) the arterial lesion from a proximal side of the arterial lesion.

The embolic protection device 120 is configured with a collapsed state and a deployed state with the deployed state of the embolic protection device 120 being shown in FIG. 1. The embolic protection device 120 assumes the collapsed state while stowed in the catheter-body lumen 112 at the distal end portion of the catheter assembly 100. The embolic protection device 120 assumes the deployed state while outside the catheter-body lumen 112. The deployed state of the embolic protection device 120 is configured with an opening such as a retrograde opening away from the catheter body 110 and toward a distal end or a distal cap of an arterial lesion. The embolic protection device 120 is configured with a basket, funnel, cone, tapered, or similar shape for capturing emboli E against blood flow. Such emboli can be dislodged from the distal end or the distal cap of the arterial lesion while crossing or ablating the arterial lesion from a proximal end or a proximal cap of the arterial lesion. The arterial lesion is shown as a chronic total occlusion ("CTO") in FIG. 1.

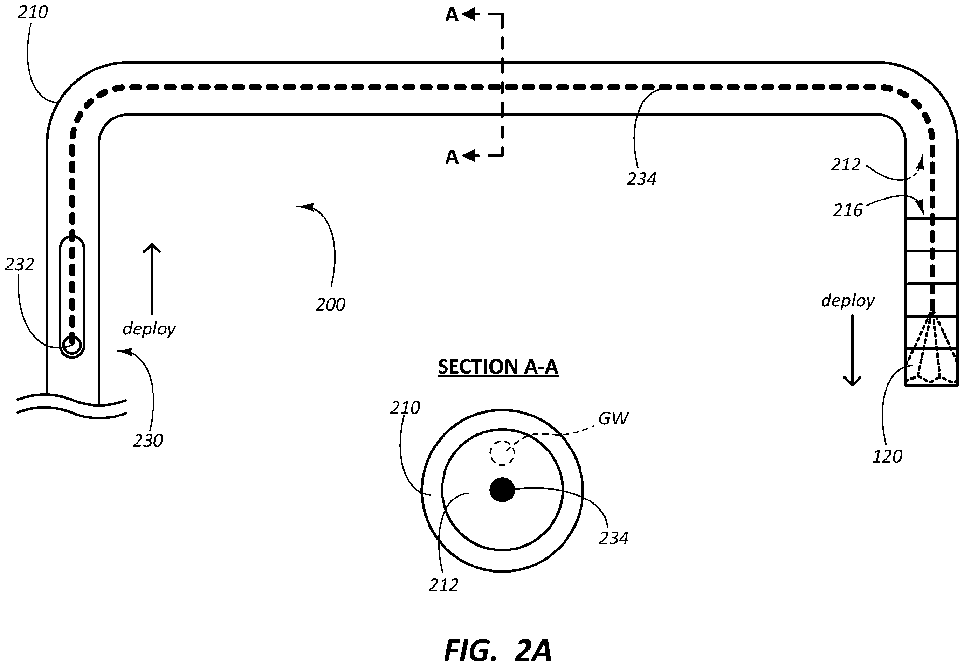

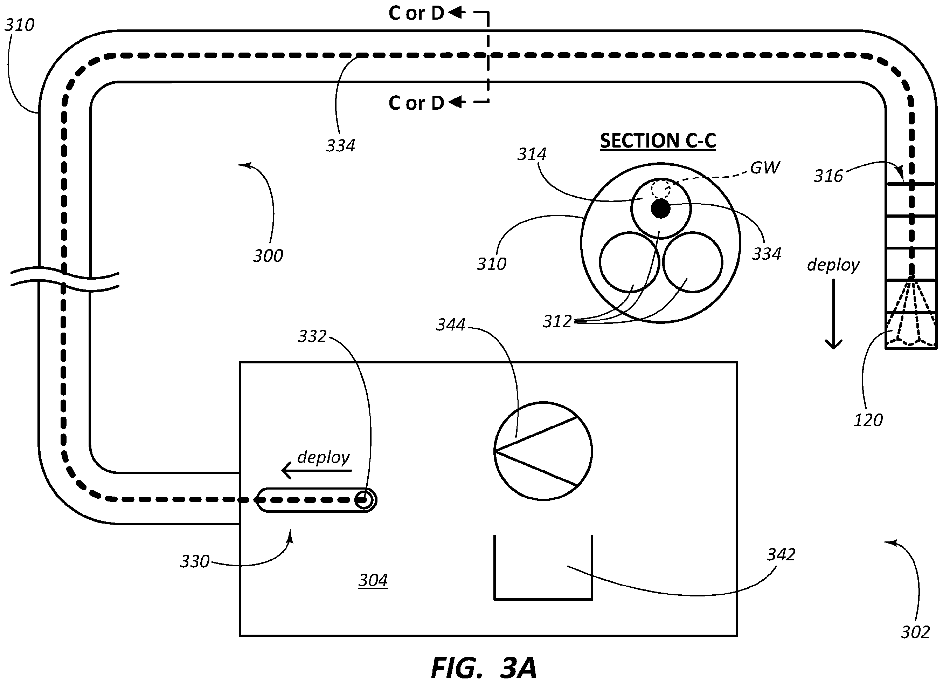

The catheter assembly 100 is configured for endoluminal advancement of the distal end portion of the catheter assembly 100 from a puncture site at a patient's artery A (e.g., the dorsalis pedis artery) toward one or more larger arteries (e.g., the anterior tibial artery, the popliteal artery, or the femoral artery). In accordance with the foregoing, such endoluminal advancement can be retrograde endoluminal advancement. The catheter assembly 100 can be advanced through the patient's vasculature over a guidewire *GW* through both the catheter body 110 and the embolic protection device 120. For example, FIGS. 2A and 3A respectively show embodiments in which the guidewire *GW* is optionally disposed in a catheter body 210 of a catheter assembly 200 and a catheter body 310 of a catheter assembly 300. Alternatively, the catheter assembly 100 can be advanced through the patient's vasculature without a guidewire *GW.* The catheter body 110 is sized accordingly. The catheter body 110 can include a diameter commensurate with an average diameter of a human dorsalis pedis artery, thereby obviating endoluminal advancement of the distal end portion of the catheter assembly 100 over a guidewire. In such embodiments, the catheter body 110 is sized at least about 1 Fr (0.333 mm), 2 Fr (0.667 mm), 3 Fr (1 mm), 4 Fr (1.333 mm), or 5 Fr (1.667 mm), optionally in tenths of the foregoing. Alternatively, the catheter body 110 is sized at no more than about 5 Fr (1.667 mm), 4 Fr (1.333 mm), 3 Fr (1 mm), 2 Fr (0.667 mm), or 1 Fr (0.333 mm), optionally in tenths of the foregoing. As such, the catheter body 110 is sized at least about 1 Fr (0.333 mm) to about 5 Fr (1.667 mm), including about 1 Fr (0.333 mm) to about 4 Fr (1.333 mm), such as about 1 Fr (0.333 mm) to about 3 Fr (1 mm), for example, about 2 Fr (0.667 mm) to about 3 Fr (1 mm). The catheter body 110 can alternatively include a diameter compatible or commensurate with an average diameter of a human fibular artery, anterior tibial artery, or posterior tibial artery for endoluminal advancement of the distal end portion of the catheter assembly 100 through the corresponding artery.

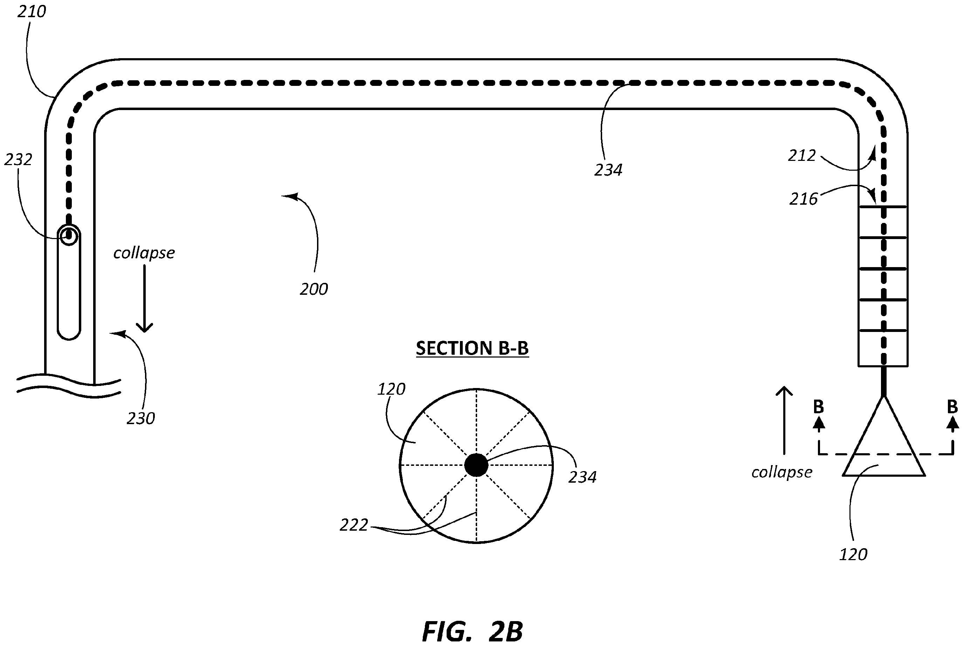

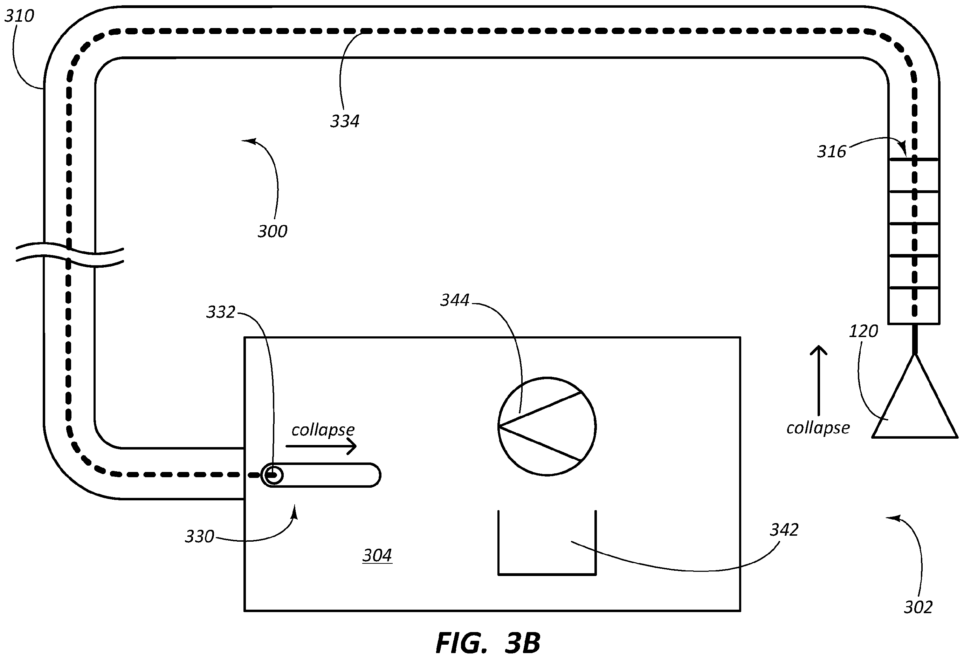

The distal end portion of the catheter assembly 100 can include one or more radiopaque markings for endoluminal advancement of the catheter assembly 100 using fluoroscopic imaging. Such radiopaque marking can be on the catheter body 110, the embolic protection device 120, or both. For example, FIGS. 2A and 2B show an embodiment in which radiopaque markings 216 are optionally around a distal end portion of a catheter body 210 of a catheter assembly 200. FIGS. 3A and 3B, too, show an embodiment in which radiopaque markings 316 are optionally around a distal end portion of a catheter body 310 of a catheter assembly 300. Alternatively or additionally, at least the distal end portion of the catheter assembly 100 can be echogenic for endoluminal advancement of the catheter assembly 100 using ultrasound.

The embolic protection device 120 can have a pliable form (e.g., a disk cut or otherwise formed from a pliable or flexible material) that facilitates the collapsed state while the embolic protection device 120 is stowed in the catheter-body lumen 112 and the deployed state while the embolic protection device 120 is outside the catheter-body lumen 112. The embolic protection device 120 can be a mesh, a porous membrane, or a non-porous membrane, optionally in one or more individually selected layers thereof. The embolic protection device 120 can be of any polymer including, but not limited to, a woven or expanded polymer such as expanded polytetrafluoroethylene ("ePTFE").

FIG. 2A provides a schematic illustrating a catheter assembly 200 including the embolic protection device 120 in the collapsed state. FIG. 2B provides a schematic illustrating the catheter assembly 200 including the embolic protection device 120 in the deployed state.

As shown, the catheter assembly 200 includes a catheter body 210 with at least one catheter-body lumen 212 from which catheter-body lumen 212 the embolic protection device 120 is deployed for capturing emboli. The catheter assembly 200 includes an insertion profile with the embolic protection device 120 in a collapsed state, and the catheter assembly 200 includes a deployment profile with the embolic protection device 120 in a deployed state. The insertion profile of the catheter assembly 200 and the collapsed state of the embolic protection device 120 is shown in FIG. 2A. The deployment profile of the catheter assembly 200 and the deployed state of the embolic protection device 120 is shown in FIG. 2B. The catheter assembly 200 further includes a control mechanism 230 configured to deploy the embolic protection device 120, thereby putting the catheter assembly 200 in the deployment profile and the embolic protection device 120 thereof in the deployed state. In some embodiments, the control mechanism 230 includes a control such as a slide button 232 or a scroll wheel at a proximal end portion of the catheter assembly 200, the control mechanism 230 connected to the embolic protection device 120 by one or more wires configured to deploy the embolic protection device 120 upon advancing the control from a stowage position to a deployment position. At least a primary wire 234 of the one or more wires is disposed in the at least one catheter-body lumen 212 as shown in Section A- A. Any other wires of the one or more wires can be auxiliary wires 222 configured to facilitate deployment of the embolic protection device 120. For example, the auxiliary wires 222 can be radially placed in or on a disk of a pliable or flexible material forming the embolic protection device 120, the radially placed auxiliary wires 222 akin to ribs of an umbrella. The auxiliary wires 222 are optionally pre-compressed to spring open the embolic protection device 120 upon deployment.

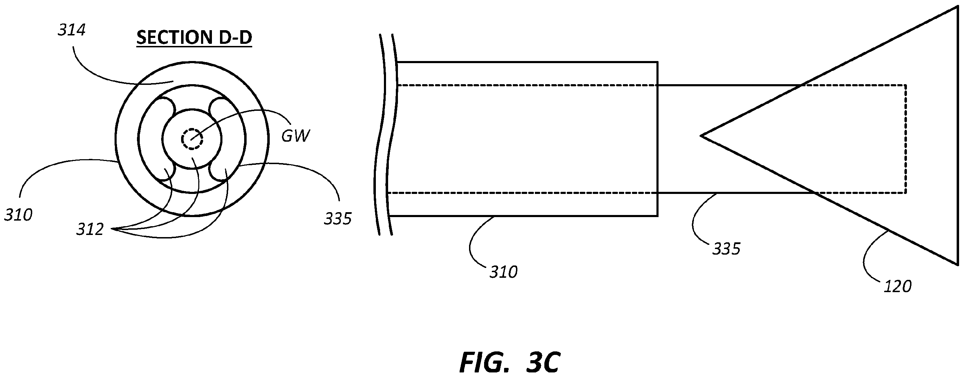

FIG. 3A provides a schematic illustrating a system 302 including a console 304 and a catheter assembly 300 with the embolic protection device 120 in the collapsed state. FIG. 3B provides a schematic illustrating the system 302 including the console 304 and the catheter assembly 300 including the embolic protection device 120 in a deployed state. FIG. 3C provides a schematic illustrating an alternative for the catheter assembly 300 of the system 302 of FIGS. 3 A and 3B.

As shown, the catheter assembly 300 includes a catheter body 310 with a number of lumens 312 including at least a primary lumen 314 from which the embolic protection device 120 is deployed for capturing emboli. When additional lumens are present, each lumen of the lumens 312 other than the primary lumen 314 is independently configured for flushing an arterial lumen around a distal end or a distal cap of an arterial lesion, aspirating emboli dislodged from the arterial lesion, or both flushing and aspirating in accordance with the foregoing. In addition to the foregoing, one of the additional lumens can be configured for a guidewire *GW* as shown in FIG. 3C. While two additional lumens are shown in Section C-C of FIG. 3A and three additional lumens are shown in Section D-D of FIG. 3C, the catheter assembly 300 can include one, two, three, or more than three additional lumens. If the catheter assembly 300 includes one additional lumen, the one additional lumen can be configured for flushing an arterial lumen around a distal end or a distal cap of an arterial lesion, aspirating emboli dislodged from the arterial lesion, or both flushing and aspirating in accordance with the foregoing. If the catheter assembly 300 includes two additional lumens, a first additional lumen can be configured for flushing an arterial lumen around a distal end or a distal cap of an arterial lesion while a second additional lumen can be configured for aspirating emboli dislodged from the arterial lesion. If the catheter assembly 300 includes three additional lumens, a first additional lumen can be configured for flushing an arterial lumen around a distal end or a distal cap of an arterial lesion, a second additional lumen can be configured for aspirating emboli dislodged from the arterial lesion, and a third additional lumen can be configured for advancing the catheter assembly over a guidewire *GW.*

The catheter assembly 300 includes an insertion profile with the embolic protection device 120 in a collapsed state, and the catheter assembly 300 includes a deployment profile with the embolic protection device 120 in a deployed state. The insertion profile of the catheter assembly 300 and the collapsed state of the embolic protection device 120 is shown in FIG. 3A. The deployment profile of the catheter assembly 300 and the deployed state of the embolic protection device 120 is shown in FIGS. 3B and 3C. As opposed to the catheter assembly 200, the console 302 includes a control mechanism 330 configured to deploy the embolic protection device 120, collapse the embolic protection device 120, or alternately deploy and collapse the embolic protection device 120. In some embodiments, the control mechanism 330 includes a control such as a slide button 332 on the console 302, the control mechanism 330 connected to the embolic protection device 120 by one or more wires or other devices configured to deploy the embolic protection device 120 upon advancing the control from a stowage position to a deployment position. At least a primary wire 334 (FIGS. 3A and 3B) of the one or more wires or other devices can be disposed in the primary lumen 314 as shown in Section C-C for deploying the embolic protection device 120 connected thereto. Alternatively, at least a tubular structure 335 (FIG. 3C) of the one or more wires or other devices can be disposed in the primary lumen 314 as shown in Section D-D for deploying the embolic protection device 120 connected thereto. The tubular structure 335 includes each lumen of the lumens 312 other than the primary lumen 314. Any other wires of the one or more wires or other devices can be auxiliary wires such as the auxiliary wire 222 shown in FIG. 2B, the auxiliary wires 222 configured to facilitate deployment of the embolic protection device 120. For example, the auxiliary wires 222 can be radially placed in or on a disk of a pliable or flexible material forming the embolic protection device 120, the radially placed auxiliary wires 222 akin to ribs of an umbrella. The auxiliary wires 222 are optionally pre-compressed to spring open the embolic protection device 120 upon deployment.

The console 302 can further include one or more pumps 344 and a reservoir 342 in some embodiments. The one or more pumps 344 are fluidly connected to at least one lumen of the additional lumens of lumens 312 for flushing the arterial lumen around a distal end or a distal cap of an arterial lesion, aspirating emboli dislodged from the arterial lesion, or both flushing and aspirating in accordance with the foregoing. The reservoir 342 is fluidly connected to at least one lumen of the additional lumens of lumens 312, the reservoir 342 configured for collecting emboli dislodged from the arterial lesion.

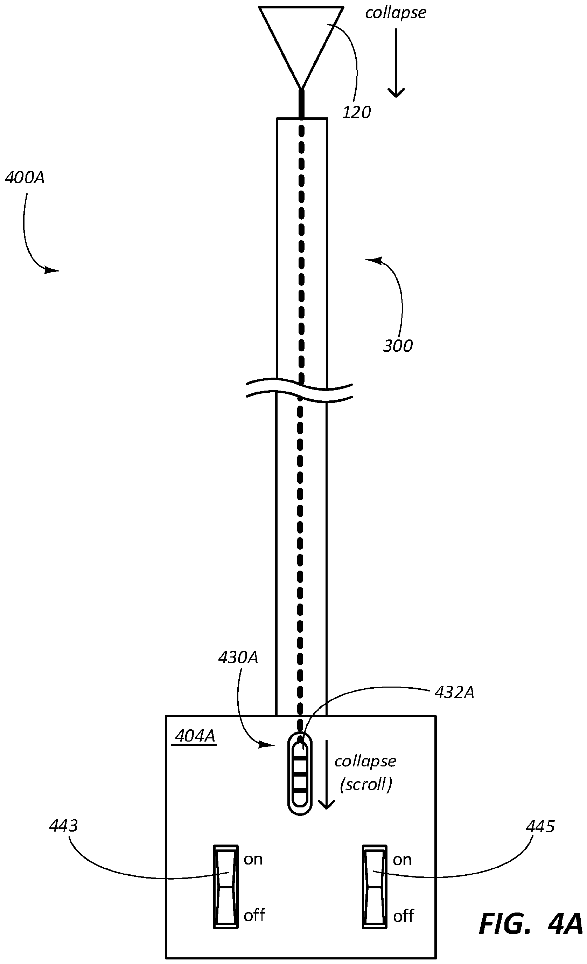

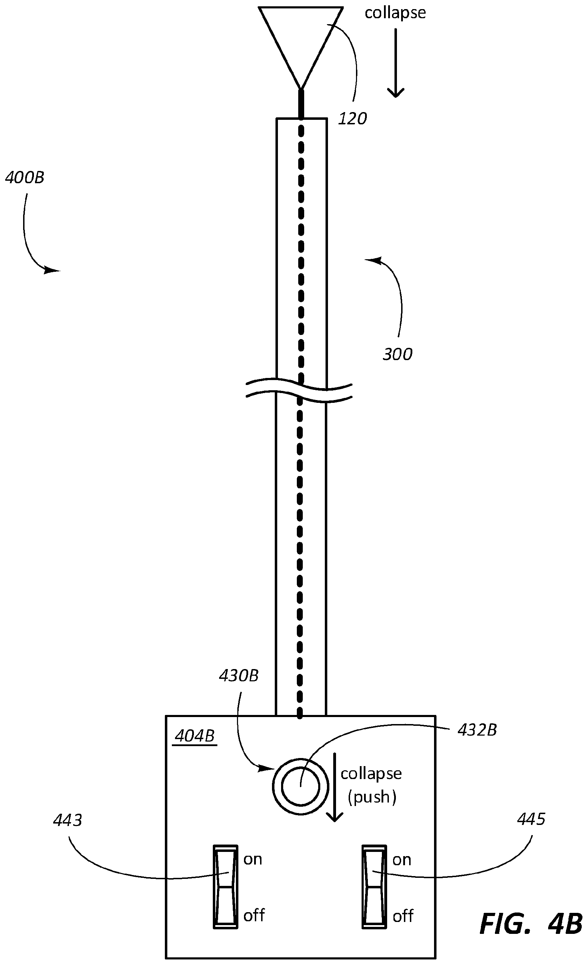

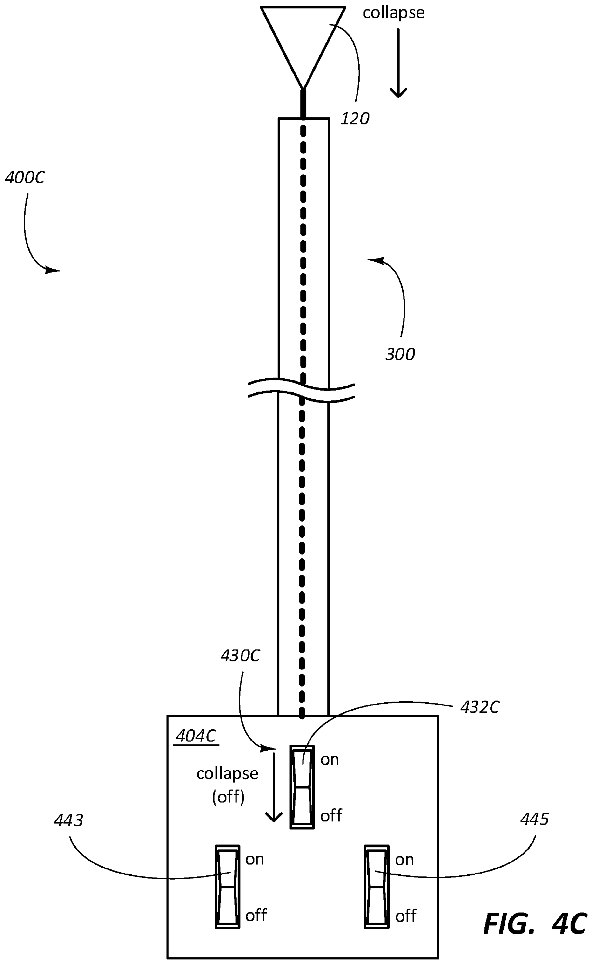

FIGS. 4A, 4B, and 4C provide schematics illustrating systems, each system of which includes a console and a different control mechanism with a different control for the embolic protection device 120, wherein each different control mechanism is an alternative to at least the control mechanism 330 of FIGS. 3A and 3B.

FIG. 4A provides a schematic illustrating a system 400A including a console 404A and a scroll wheel-based control mechanism 430A with a scroll wheel 432A for the control. The control mechanism 430A is configured to deploy the embolic protection device 120, collapse the embolic protection device 120, or alternately deploy and collapse the embolic protection device 120 by scrolling the scroll wheel 432A. The scroll wheel-based control mechanism 430A can be purely mechanical or include electrical components as well, for example, for electrically fine tuning a speed ratio of the scroll wheel-based control mechanism 430A. When purely mechanical, the scroll wheel-based control mechanism 430A can be used in place of the control mechanism 230 of the catheter assembly 200. (*See* FIGS. 2A and 2B.)

FIG. 4B provides a schematic illustrating a system 400B including a console 404B and a push button-based control mechanism 430B with a push button 432B for the control. The control mechanism 430B is configured to deploy the embolic protection device 120, collapse the embolic protection device 120, or alternately deploy and collapse the embolic protection device 120 by pushing the push button 432B.

FIG. 4C provides a schematic illustrating a system 400C including a console 404C and a switch-based control mechanism 430C with a switch 432C for the control. The control mechanism 430C is configured to deploy the embolic protection device 120, collapse the embolic protection device 120, or alternately deploy and collapse the embolic protection device 120 by flipping the switch 432C.

As shown in FIGS. 4A, 4B, and 4C, each console of the consoles 400A, 400B, and 400C can include one or more controls such as switch 343 and switch 345 configured to switch corresponding pumps of the one or more pumps 344 on and off. For example, the switch 343 can be configured to turn a first pump on and off, the first pump configured to supply a liquid such as water, saline, heparinized saline, or the like when switched on for flushing an arterial lumen around a distal end or a distal cap of an arterial lesion. For example, the switch 345 can be configured to turn a second pump on and off, the second pump configured to supply a vacuum when switched on for aspirating the arterial lumen around the distal end or the distal cap of the arterial lesion. Other controls for the one or more pumps 344 include, but are not limited to, dials, slide buttons such as the slide button 332 of the control mechanism 330, and push buttons such as the push button 432B of the control mechanism 430B.

Currently, when surgically intervening to treat peripheral artery disease, an arterial lesion such as a CTO is crossed with a crossing device for balloon angioplasty and stenting or ablated with an atherectomy device, each surgical intervention of which can release emboli. In any patient, particularly those with already reduced vascular function or a risk for a large amount of embolic material, there is a desire to capture the emboli. In cases where the arterial lesion is a CTO, there is no way to place guidewire-based distal protection devices until the CTO has already been crossed, which can lead to a large amount of embolic material. However, embolic protection devices of the catheter assemblies provided herein can be placed distal to an arterial lesion by accessing an artery at a puncture site distal to the arterial lesion, thereby obviating crossing the arterial lesion to place an embolic protection device.

A method of placing an embolic protection device of a catheter assembly provided herein and capturing emboli therein includes advancing a distal end portion of the catheter assembly through an arterial lumen to a distal end or a distal cap of an arterial lesion, the catheter assembly including the embolic protection device stowed in the distal end portion; deploying the embolic protection device from the distal end portion of the catheter assembly, the embolic protection device including a retrograde opening; and capturing any emboli dislodged from the arterial lesion in the embolic protection device. Again, the method obviates crossing the arterial lesion from a proximal end or a proximal cap of the arterial lesion to place the embolic protection device at the distal end or the distal cap of the arterial lesion.

Before advancing the distal end portion of the catheter assembly through the arterial lumen to the distal end or the distal cap of the arterial lesion, a first puncture site is created by a puncture in an artery including the arterial lumen. The puncture can be a puncture in a smaller sized artery for advancing the distal end portion of the catheter assembly toward one or more larger sized arteries. For example, the puncture can be a retrograde puncture in the dorsalis pedis artery for advancing the distal end portion of the catheter assembly toward the anterior tibial artery, the popliteal artery, or the femoral artery. Alternatively, the puncture can be a retrograde puncture at the anterior tibial artery for advancing the distal end portion of the catheter assembly toward the popliteal artery or the femoral artery. Alternatively, the puncture can be a retrograde puncture at the fibular artery for advancing the distal end portion of the catheter assembly toward the posterior tibial artery, the popliteal artery, or the femoral artery. Alternatively, the puncture can be a retrograde puncture at the posterior tibial artery for advancing the distal end portion of the catheter assembly toward the popliteal artery or the femoral artery.

In addition to placing the embolic protection device of the catheter assembly at the distal end or the distal cap of the arterial lesion, the method includes advancing a recanalizing device (e.g., a device for crossing or ablating the arterial lesion) through the arterial lumen to a proximal end or a proximal cap of the arterial lesion for an antegrade recanalization procedure (e.g., balloon angioplasty, atherectomy, etc.). The emboli dislodged from the arterial lesion include emboli dislodged during the recanalization procedure.

Before advancing the recanalizing device through the arterial lumen to the proximal end or the proximal cap of the arterial lesion, a second puncture site is created by a puncture in an artery including the arterial lumen. The puncture can be a puncture in a larger sized artery for advancing the recanalizing device toward one or more smaller sized arteries. For example, the puncture can be an antegrade or a retrograde puncture in the femoral artery for advancing the recanalizing device toward the dorsalis pedis artery or the fibular artery, wherein the antegrade puncture can be for intraluminal recanalization and the retrograde puncture can be for subintimal recanalization.

While protection at the distal side of arterial lesions is largely described herein, protection at the distal side of veinal lesions is also possible with the embolic protection devices provided herein, as well as the systems and methods thereof.

Some particular embodiments have been provided herein, and while the particular embodiments have been provided in some detail. Additional adaptations and/or modifications can appear to those of ordinary skill in the art. Departures may be made from the particular embodiments provided herein without departing from the scope of the claims which follow.

## Claims

1. A catheter assembly (300) for capturing emboli (E) dislodged from an arterial lesion, the assembly comprising:
a catheter body (310) including a lumen (314), the catheter body sized for endoluminal advancement of a distal end portion of the catheter assembly from a puncture site at a human artery (A) selected from a dorsalis pedis artery, a fibular artery, an anterior tibial artery, and a posterior tibial artery toward one or more larger arteries and toward a distal end of the arterial lesion;
an insertion profile and a deployment profile; and
an embolic protection device (120) configured with:
a collapsed state when stowed in the lumen at the distal end portion of the catheter assembly in the insertion profile; and
a deployed state when outside the lumen in the deployment profile, the deployed state configured with a retrograde opening away from the catheter body and toward a distal end or distal cap of an arterial lesion for capturing emboli (E) dislodged from the arterial lesion; and
wherein the catheter body further includes one or more additional lumens (312), each lumen of the one or more additional lumens independently configured for flushing an arterial lumen around a distal end of an arterial lesion, aspirating emboli dislodged from an arterial lesion, or both flushing and aspirating in accordance with the foregoing.

2. The catheter assembly of claim 1,
wherein the catheter body is sized at 3 Fr or less.

3. The catheter assembly of either claim 1 or 2,
wherein the embolic protection device (120) includes a mesh or a porous membrane.

4. The catheter assembly of either claim 1 or 2,
wherein the embolic protection device (120) includes non-porous membrane.

5. The catheter assembly of any claim of claims 1-4, further comprising:
a control mechanism (330) configured to deploy the embolic protection device.

6. The catheter assembly of claim 5,
wherein the control mechanism includes a control selected from a slide button (332) and a scroll wheel (432A) at a proximal end portion of the catheter assembly, the control mechanism connected to the embolic protection device by one or more wires (334) configured to deploy the embolic protection device upon advancing the control to a deployment position.

7. The catheter assembly of any claim of claims 1-6,
wherein the catheter assembly is configured for endoluminal advancement of the distal end portion of the catheter assembly over a guidewire (GW).

8. The catheter assembly of any claim of claims 1-6,
wherein the catheter body includes a diameter commensurate with an average diameter of a human dorsalis pedis artery, the diameter obviating endoluminal advancement of the distal end portion of the catheter assembly over a guidewire.

9. The catheter assembly of any claim of claims 1-8,
wherein the distal end portion of the catheter assembly includes one or more radiopaque markings (316) for endoluminal advancement of the catheter assembly using fluoroscopic imaging.

10. The catheter assembly as claimed in any one of the preceding claims, wherein the deployed state of the embolic protection device (120) takes on the shape of a basket, funnel or cone such that the cross-sectional area of the embolic protection device decreases from the opening to the catheter body following deployment.

11. A catheter system, comprising:
the catheter assembly of any one of the preceding claims, and:
a console (304) configured to control one or more functions of the catheter assembly.

12. The catheter system of claim 11, the console further comprising:
one or more pumps (344) fluidly connected to at least one of the one or more additional lumens for flushing an arterial lumen around a distal end of an arterial lesion, aspirating emboli dislodged from an arterial lesion, or both flushing and aspirating; and
a reservoir (342) configured for collecting emboli dislodged from an arterial lesion.

13. The catheter system of claim 11 or 12, including a recanalizing device advanceable through the lumen of the said human artery.

## Patentansprüche

1. Katheteranordnung (300) zum Einfangen von Embolien (E), die sich von einer arteriellen Läsion gelöst haben, wobei die Anordnung umfasst:
einen Katheterkörper (310), der ein Lumen (314) einschließt, wobei der Katheterkörper bemessen ist für endoluminales Vorschieben eines distalen Endabschnitts der Katheteranordnung von einer Punktionsstelle an einer menschlichen Arterie (A), die aus einer Arteria dorsalis pedis, einer Arteria fibularis, einer Arteria tibialis anterior und einer Arteria tibialis posterior ausgewählt ist, zu einer oder mehreren größeren Arterien und zu einem distalen Ende der arteriellen Läsion;
ein Einführungsprofil und ein Einsatzprofil; und
eine Embolieschutzvorrichtung (120), die mit Folgendem konfiguriert ist:
einem kollabierten Zustand, wenn sie in dem Lumen an dem distalen Endabschnitt der Katheteranordnung in dem Einführungsprofil verstaut ist; und
einem eingesetzten Zustand, wenn sie sich außerhalb des Lumens im Einsatzprofil befindet, wobei der eingesetzte Zustand mit einer retrograden Öffnung vom Katheterkörper weg und zu einem distalen Ende oder einer distalen Kappe einer arteriellen Läsion hin konfiguriert ist, um von der arteriellen Läsion abgelöste Embolien (E) einzufangen, und
wobei der Katheterkörper weiter ein oder mehrere zusätzliche Lumen (312) einschließt, wobei jedes Lumen des einen oder der mehreren zusätzlichen Lumen unabhängig konfiguriert ist, um gemäß dem Vorstehenden ein arterielles Lumen um ein distales Ende einer arteriellen Läsion herum zu spülen, von einer arteriellen Läsion abgelöste Embolien abzusaugen oder sowohl zu spülen als auch abzusaugen.

2. Katheteranordnung nach Anspruch 1,
wobei der Katheterkörper eine Größe von 3 Fr oder weniger aufweist.

3. Katheteranordnung nach Anspruch 1 oder 2,
wobei die Embolieschutzvorrichtung (120) ein Netz oder eine poröse Membran einschließt.

4. Katheteranordnung nach Anspruch 1 oder 2,
wobei die Embolieschutzvorrichtung (120) eine nicht poröse Membran einschließt.

5. Katheteranordnung nach einem der Ansprüche 1-4 weiter umfassend:
einen Steuermechanismus (330), der zum Einsetzen der Embolieschutzvorrichtung konfiguriert ist.

6. Katheteranordnung nach Anspruch 5,
wobei der Steuermechanismus eine Steuereinheit einschließt, die aus einem Schiebeknopf (332) und einem Scrollrad (432A) an einem proximalen Endabschnitt der Katheteranordnung ausgewählt ist, wobei der Steuermechanismus mit der Embolieschutzvorrichtung durch einen oder mehrere Drähte (334) verbunden ist, die so konfiguriert sind, dass sie die Embolieschutzvorrichtung einsetzen, wenn die Steuereinheit zu einer Einsatzposition vorgeschoben wird.

7. Katheteranordnung nach einem der Ansprüche 1-6,
wobei die Katheteranordnung für endoluminales Vorschieben des distalen Endabschnitts der Katheteranordnung über einen Führungsdraht (GW) konfiguriert ist.

8. Katheteranordnung nach einem der Ansprüche 1-6,
wobei der Katheterkörper einen Durchmesser einschließt, der einem durchschnittlichen Durchmesser einer menschlichen Arteria dorsalis pedis entspricht, wobei der Durchmesser endoluminales Vorschieben des distalen Endabschnitts der Katheteranordnung über einen Führungsdraht verhindert.

9. Katheteranordnung nach einem der Ansprüche 1-8,
wobei der distale Endabschnitt der Katheteranordnung eine oder mehrere röntgendichte Markierungen (316) zum endoluminalen Vorschieben der Katheteranordnung unter Verwendung fluoroskopischer Bildgebung einschließt.

10. Katheteranordnung nach einem der vorstehenden Ansprüche, wobei die Embolieschutzvorrichtung (120) im eingesetzten Zustand die Form eines Korbes, Trichters oder Kegels annimmt, so dass sich die Querschnittsfläche der Embolieschutzvorrichtung nach dem Einsatz von der Öffnung zum Katheterkörper hin verringert.

11. Kathetersystem, das Folgendes umfasst:
die Katheteranordnung nach einem der vorstehenden Ansprüche, und:
eine Konsole (304), die zum Steuern einer oder mehrerer Funktionen der Katheteranordnung konfiguriert ist.

12. Kathetersystem nach Anspruch 11, wobei die Konsole weiter umfasst:
eine oder mehrere Pumpen (344), die mit mindestens einem des einen oder der mehreren zusätzlichen Lumen in Fluidverbindung stehen, um ein arterielles Lumen um ein distales Ende einer arteriellen Läsion zu spülen, von einer arteriellen Läsion abgelöste Embolien abzusaugen oder sowohl zu spülen als auch abzusaugen; und
einen Behälter (342), der zum Sammeln von Embolien konfiguriert ist, die sich von einer arteriellen Läsion gelöst haben.

13. Kathetersystem nach Anspruch 11 oder 12, einschließlich einer rekanalisierenden Vorrichtung, die durch das Lumen der menschlichen Arterie vorgeschoben werden kann.

## Revendications

1. Ensemble (300) cathéter pour capturer des emboles (E) délogés d'une lésion artérielle, l'ensemble comprenant :
un corps (310) de cathéter incluant une lumière (314), le corps de cathéter étant dimensionné pour l'avancée endoluminale d'une partie d'extrémité distale de l'ensemble cathéter à partir d'un site de perforation au niveau d'une artère (A) humaine choisie parmi une artère dorsale du pied, une artère fibulaire, une artère tibiale antérieure et une artère tibiale postérieure vers une ou plusieurs artères plus grandes et vers une extrémité distale de la lésion artérielle ;
un profil d'insertion et un profil de déploiement ; et
un dispositif (120) de protection embolique configuré avec :
un état replié lorsqu'il est mis en place dans la lumière au niveau de la partie d'extrémité distale de l'ensemble cathéter dans le profil d'insertion : et
un état déployé lorsqu'il est à l'extérieur de la lumière dans le profil de déploiement, l'état déployé étant configuré avec une ouverture rétrograde à l'opposé du corps de cathéter et vers une extrémité distale ou une coiffe distale d'une lésion artérielle pour capturer des emboles (E) délogés de la lésion artérielle, et
dans lequel le corps de cathéter inclut en outre une ou plusieurs lumières (312) additionnelles, chaque lumière des une ou plusieurs lumières additionnelles étant configurée indépendamment pour rincer une lumière artérielle autour d'une extrémité distale d'une lésion artérielle, aspirer des emboles délogés d'une lésion artérielle, ou à la fois pour un rinçage et une aspiration conformément à ce qui précède.

2. Ensemble cathéter selon la revendication 1,
dans lequel le corps de cathéter est dimensionné à 3 Fr ou moins.

3. Ensemble cathéter selon la revendication 1 ou la revendication 2,
dans lequel le dispositif (120) de protection embolique inclut une maille ou une membrane poreuse.

4. Ensemble cathéter selon la revendication 1 ou la revendication 2,
dans lequel le dispositif (120) de protection embolique inclut une membrane non poreuse.

5. Ensemble cathéter selon l'une quelconque des revendications 1 à 4, comprenant en outre :
un mécanisme (330) de commande configuré pour déployer le dispositif de protection embolique.

6. Ensemble cathéter selon la revendication 5,
dans lequel le mécanisme de commande inclut une commande choisie parmi un bouton coulissant (332) et une molette (432A) au niveau d'une partie d'extrémité proximale de l'ensemble cathéter, le mécanisme de commande relié au dispositif de protection embolique par un ou plusieurs fils (334) configurés pour déployer le dispositif de protection embolique lors de l'avancée de la commande vers une position de déploiement.

7. Ensemble cathéter selon l'une quelconque des revendications 1 à 6,
dans lequel l'ensemble cathéter est configuré pour l'avancée endoluminale de la partie d'extrémité distale de l'ensemble cathéter sur un fil-guide (GW).

8. Ensemble cathéter selon l'une quelconque des revendications 1 à 6,
dans lequel le corps de cathéter inclut un diamètre correspondant à un diamètre moyen d'une artère dorsale du pied humaine, le diamètre empêchant l'avancée endoluminale de la partie d'extrémité distale de l'ensemble cathéter sur un fil-guide.

9. Ensemble cathéter selon l'une quelconque des revendications 1 à 8,
dans lequel la partie d'extrémité distale de l'ensemble cathéter inclut un ou plusieurs marquages (316) radio-opaques pour l'avancée endoluminale de l'ensemble cathéter à l'aide d'une imagerie fluoroscopique.

10. Ensemble cathéter selon l'une quelconque des revendications précédentes, dans lequel l'état déployé du dispositif (120) de protection embolique prend la forme d'un panier, d'un entonnoir ou d'un cône de telle manière que l'aire de la section transversale du dispositif de protection embolique diminue de l'ouverture au corps de cathéter après le déploiement.

11. Système de cathéter, comprenant :
l'ensemble cathéter selon l'une quelconque des revendications précédentes, et :
une console (304) configurée pour commander une ou plusieurs fonctions de l'ensemble cathéter.

12. Système de cathéter selon la revendication 11, la console comprenant en outre :
une ou plusieurs pompes (344) reliées de manière fluidique à au moins une des une ou plusieurs lumières additionnelles pour rincer une lumière artérielle autour d'une extrémité distale d'une lésion artérielle, aspirer des emboles délogés d'une lésion artérielle, ou à la fois pour un rinçage et une aspiration ; et
un réservoir (342) configuré pour collecter des emboles délogés d'une lésion artérielle.

13. Système de cathéter selon la revendication 11 ou la revendication 12, incluant un dispositif de recanalisation pouvant être avancé à travers la lumière de ladite artère humaine.
